# EUROPEAN PATENT APPLICATION

(11) **EP 1 437 415 A1**
(43) Date of publication of application: **14.07.2004**
(21) Application number: 03100042.5
(22) Date of filing: 13.01.2003
(51) Int. Cl.: C12P 7/56

(54) **Preparation of lactic acid from a pentose-containing substrate**

(71) Applicant: PURAC Biochem BV, 4206 AC Gorinchem (NL)
(72) Inventor: Otto, Roel, 4201 BC, GORINCHEM (NL)
(74) Representative: Beetz, Tom

(57) **Abstract**

This invention relates to the production of lactic acid and/or lactate from pentose-containing substrate, particularly from xylose-containing substrate
Currently, lactic acid is commercially produced from glucose, starch, liquefied starch or sucrose. At present, these substrates are the most important contributor to the manufacturing cost price of lactic acid. Lignocellulosic biomass offers a cost attractive alternative as a substrate for the biological production of lactic acid because it is readily available, has no competing food value, and is less expensive than either starch or sucrose.
We have found that moderately thermophilic *Bacillus* species are able to ferment pentoses, more specifically xylose to enantiomerically pure lactic acid and/or lactate. Said conversion of pentoses leads to virtually only C₃ compounds, i.e. said conversion runs via a homofermentive pathway, which C₃ compounds can be recovered as lactic acid and/or lactate.

## Description

### FIELD OF THE INVENTION

This invention relates to the production of lactic acid from pentose-containing substrate, particularly from xylose-containing substrate.

### BACKGROUND OF THE INVENTION

Lactic acid, and its salts known as lactate, are commercially viable products useful in various fields including medicine, biodegradable polymers and food processing. Currently, lactic acid is commercially produced from glucose, starch, liquefied starch or sucrose. At present, these substrates are an important contributor to the manufacturing cost price of lactic acid. Lignocellulosic biomass offers a cost attractive alternative as a substrate for the biological production of lactic acid because it is readily available, has no competing food value, and is less expensive than either starch or sucrose. Theoretically, a microorganism would be able to ferment the sugars contained in the biomass to lactic acid. However, several obstacles preclude efficient utilization of this feedstock by a microorganism for lactic acid production. Lignocellulosic substrates are largely composed of cellulose, hemicellulose and lignin. While several microorganisms can efficiently ferment the glucose component in cellulose, conversion of the pentose sugars contained in the hemicellusose fraction of biomass has proven more difficult. The most abundant pentose sugars in hemicellulose include D-xylose and L-arabinose. Fermentation of xylose and arabinose remains a major obstacle for economical conversion of plant-originated biomass.

Many heterolactic and facultative heterolactic lactic acid bacteria are able to ferment pentoses. The metabolic route used by these organisms to ferment these sugars is simple: a pentose e.g. D-xylose (aldose) enters the cell where it is isomerised to xylulose (ketose) and subsequently phosphorylated at the cost of 1 ATP to yield xylulose-5-phosphate, which is then cleaved into glyceraldehyde-3-phosphate and acetyl-phosphate by phosphoketolase (EC 4.1.2.9). This metabolic pathway is known as the phosphoketolase pathway (Lengeler,J.W.; G.Drews; H.G.Schlegel, Biology of prokaryotes, 1999, Thieme Verlag, Stuttgart, Germany). The glyceraldehyde-3-phosphate that is produced in the phosphoketolase reaction is converted to pyruvic acid as in the Emden-Meyerhof pathway yielding 2 ATP and 1 NADH₂ (Lengeler,J.W.; G.Drews; H.G.Schlegel, Biology of prokaryotes, 1999,Thieme Verlag, Stuttgart, Germany) Pyruvic acid is finally reduced with NADH₂ to lactic acid. The acetyl-phosphate that is produced in the phosphoketolase reaction is converted by acetate kinase (EC 2.7.2. 1) to acetate with the formation of 1 ATP. In the course of the fermentation of a pentose, 1 NADH₂ is formed and consumed; the net ATP yield is 2 per mol pentose.
Heterofermentative lactic acid bacteria use a similar pathway for the fermentation of hexoses. A hexose e.g. glucose is first phosphorylated to glucose-6-phosphate, oxidised to yield 6-phosphogluconate and finally oxidatively decarboxylated to yield ribulose-5-phosphate and carbon dioxide. Epimerisation of ribulose-5-phosphate yields xylulose-5-phosphate, which enters the phosphoketolase pathway. Contrary to the fermentation of pentoses the fermentation of hexoses by heterofermentative lactic acid bacteria produces an excess of reducing power (3 NADH₂) which is used to reduce acetyl-phosphate to ethanol and pyruvic acid to lactic acid. In this scheme no acetic acid is produced from acetyl-phosphate, hence the ATP yield of the fermentation of hexoses is only half that of the fermentation of pentoses; 1 ATP per mol hexose fermented.

In the above metabolic pathway of pentose fermentation the enzyme phosphoketolase plays a fate-determining role because it is this enzyme that breaks up the C₅ carbon skeleton of pentoses into a C₃ moiety, which finally can be recovered as lactic acid and a C₂ moiety, which ends up as acetic acid. For the production of lactic acid, which understandably is geared towards maximal lactate yield the formation of acetic acid is wasteful. A small number of reports, however, indicate that some *Lactobacillus* species e.g. *Lactobacillus* species MONT4 ferment certain pentoses almost exclusively to lactic acid ( Barre P., *Identification of thermobacteria and homofermentative, thermophilic pentose utilizing Lactobacilli from high temperature fermenting grape must,* J. Appl. Bacteriol. 1978, 44, 125 - 129). In *Lactobacillus* species MONT4, pentoses are dissimilated by a pathway, which does not involve phosphoketolase, but by a metabolic pathway that involves transaldolase (EC 2.2.1.2) and transketolase (EC 2.2.1.1) (US 5,798,237). This pathway is known as the transaldolase/transketolase pathway.

The higher lactate yield on pentoses of this pathway, however, comes at a price for the organism. Whilst the ATP yield of the phosphoketolase pathway is 2 per mol of pentose that of the transaldolase / transketolase pathway is 5 ATP per 3 moles of pentose. This lower ATP yield may be one of the reasons why lactic acid bacteria with a homolactic pattern of pentose fermentation are relatively rare. From an industrial point of view it is relevant to note here that *Lactobacillus* species MONT4 is unable to ferment xylose. Recently *Lactobacillus* species MONT4, was genetically engineered with xylose isomerase and xylulokinase genes from *Lactobacillus pentosus* to impart to this organism the ability to ferment xylose. This has been described in US 5,798,237.

Although micro organisms such as *Lactobacillus* species are producers of lactic acid, certain properties make these organisms less suitable for the industrial manufacture of lactic acid: *Lactobacillus* species require a fair amount of organic nitrogen in the fermentation medium, as well as growth promoting substances, so that the broth becomes more expensive and the lactic acid more difficult to purify when a simple fementation medium can be used. Furthermore many *Lactobacillus* species, *Lactobacillus* sp MONT4 included produce lactic acid with a low enantiomeric purity (see: Barre,P. Identification of thermobacteria and homofermentative, thermophilic pentose utilizing Lactobacilli from high temperature fermenting grape must. J. Appl. Bacteriol. 1978, 44, 125 - 129). It is one of the objects of this invention to provide a method, which is devoid of these disadvantages.

We have now found that naturally occuring moderately thermophilic *Bacillus* species are able to ferment pentoses, more specifically xylose, predominantly to enantiomerically pure lactic acid and/or lactate. Said conversion of pentoses leads to virtually only C₃ compounds, i.e. said conversion runs via a homofermentative, which C₃ compounds can be recovered as lactic acid and/or lactate. Moderately thermophilic *Bacillus* species are bacterial strains which are capable of growing at temperatures between 30-65 °C. Examples hereof are *Bacillus coagulans, Bacillus smithii, Geobacillus stearothermophilus* and *Bacillus thermoamylovorans.* These types of microorganisms are nutritionally less demanding than *Lactobacilli.* An additional advantage of these types of microorganisms, is that the higher growth temperatures (*Lactobacillus* species have growing temperatures of at most 50 °C) make it easier to avoid infections in industrial scale fermentation systems. Hence, the present invention is directed to a process for the preparation of lactic acid wherein a pentose-containing substrate is fermented by a moderately thermophilic *Bacillus* species.

The choice of substrates will depend on cost and supply of the substrate to be fermented to lactic acid and/or lactate. A typical low-cost supply of pentoses is from hemicellulose. Xylose, arabinose and other pentoses are liberated from hemicellulosic materials by treatment with steam and/or an acid or alkali. Smaller amounts of other sugars such as glucose are also separated during this treatment and are also fermented by the moderately thermophilic *Bacillus* species to lactic acid and/or lactate.

Lignocellulosic substrates comprise both cellulose, hemicellulose and lignine. These types of substrates may be made accessible for hydrolysation by steam and/or mild acid or alkali treatment. When the substrate comprises cellulosic material, the cellulose may be hydrolysed to sugars simultaneously or separately and also fermented to lactic acid. Since hemicellulose is generally easier to hydrolyse to sugars than cellulose, it is preferable to first prehydrolyse the hemicellulosic material, separate the soluble pentose sugars and then hydrolyse the cellulose. Hydrolysation may be done enzymatically (with cellulase for celluloses and hemicellulase for hemicellulose) or chemically by acid treatment. Both pentose and hexose sugars may be simultaneously or separately fermented to lactic acid and/or lactate using the moderately thermophilic *Bacillus* species. If so desired, the hexoses may be fermented by a different microorganism to lactic acid and/or lactate i.e. in mixed culture with e.g. yeasts, fungi or other known lactic acid-producing bacteria such as *Lactobacillus* species and *Bacillus* species differing from the ones used for the pentose fermentation.

The fermentation conditions to form lactic acid and/or lactate are known per se and are described in WO 01/27064, W099/19290, and WO 98/15517. Accordingly, the temperature may range from 0 to 80 °C, while the pH (which decreases upon lactic acid formation) ranges from 3 to 8. A pH below 5 is generally desirable, as part of the lactic acid formed will then be present in its free-acid form instead of in its salt form. Furthermore, at low pH there is less risk of contamination with other micro organisms. Any of the many known types of apparatus may be used for the fermentation according to the present invention.

The microorganism according to the present invention may be used as a biologically pure culture or it may be used with other lactic acid producing microorganisms in mixed culture. Biologically pure cultures are generally easier to optimise but mixed cultures may be able to utilise additional substrates. One may also add enzyme(s) to the fermentation vessel to aid in the degradation of substrates or to enhance lactic acid production. For example, cellulase may be added to degrade cellulose to glucose simultaneously with the fermentation of glucose to lactic acid by microorganisms. Likewise, a hemicellulase may be added to degrade hemicellulose. As mentioned-above, said hydrolysation (optionally by means of enzymes) may also be conducted prior to fermentation.

The moderately thermophilic *Bacillus* species-containing fermentation broth cultures are relatively resistant to contamination by other microorganisms. Nonetheless, it is preferred to eliminate or disable pre-existing deleterious microorganisms in the substrate added to the moderately thermophilic *Bacillus* species. This may be done by conventional techniques like filtration, pasteurisation and sterilisation.

After fermentation, the lactic acid and/or lactate is separated from the fermentation broth by any of the many conventional techniques known to separate lactic acid and/or lactate from aqueous solutions. Particles of substrate or microorganisms (the biomass) may be removed before separation to enhance separation efficiency. Said separation may be conducted by means of centrifuging, filtration, flocculation, flotation or membrane filtration. This is for instance known from WO 01/38283 wherein a continuous process for the preparation of lactic acid by means of fermentation is described.
While the discussion of the fermentation in this specification generally refers to a batch process, parts or all of the entire process may be performed continuously. To retain the microorganisms in the fermenter, one may separate solid particles from the fermentation fluids. Alternatively, the microorganisms may be immobilized for retention in the fermenter or to provide easier separation.

After separation of the lactic acid and/or lactate from the fermentation broth, the product may be subjected to one or more purification steps such as extraction, distillation, crystallisation, filtration, treatment with activated carbon etcetera. The various residual streams may be recycled, optionally after treatment, to the fermentation vessel or to any previously performed purification step.

The present invention is further illustrated by the following examples, which are not to be construed as being limitative.

### EXAMPLE 1

A culture of *Bacillus coagulans* (strain DSM 2314 from the German Culture Collection) was grown in xylose broth of the following composition:
10 g/l yeast extract,
5 g/l di-ammoniumhydrogenphosphate,
10 g/l BIS-TRIS (bis[2-hydroxymethyl]iminotris[hydroxymethyl]-methane)
3% (w/v) D-xylose (200 mmole).

The pH of the broth was adjusted to 6.6 with N HCl. The culture broth was filter sterilised (0.45 micrometers pore diameter) and distributed in 20 ml quantities in sterile screw capped tubes. The tubes were inoculated with the appropriate cultures from glycerol stocks and incubated at 46 °C. The final pH was 4.6. The culture was analysed for lactic - and acetic acid. *Bacillus coagulans* DSM 2314 produced 52 mM (mmole /liter) L(+) lactic acid and only 2 mM acetic acid.

## Claims

1. Process for the preparation of lactic acid and/or lactate wherein a pentose- containing substrate is fermented by a moderately thermophilic *Bacillus* species.

2. Process according to claim 1 wherein the pentose-containing substrate comprises xylose.

3. Process according to claim 1 or 2, wherein the moderately thermophilic *Bacillus* species is selected from *Bacillus coagulans, Bacillus smithii, Geobacillus stearothermophilus* and *Bacillus thermoamylovorans.*

4. Process according to any one of the preceding claims wherein the pentose-containing substrate comprises arabinose.

5. Process according to any one of the preceding claims wherein the substrate comprises glucose.

6. Process according to any one of the preceding claims wherein the fermentation is performed by a mixture of moderately thermophilic *Bacillus* species and another lactic-acid producing microorganism.

7. Process according to any one of the preceding claims wherein the lactic acid and/or lactate formed is separated from the fermentation broth.

8. Process according to claim 9 wherein the biomass is removed from the fermentation broth prior to the separation of the lactic acid and/or lactate therefrom.

9. Process according to any one of the preceding claims 7-8 wherein the lactic acid and/or lactate produced is subjected to one or more purification steps after separation from the fermentation broth.
